# FASCICULE DE BREVET EUROPEEN

(11) **EP 0 722 325 B1**
(45) Date de publication et mention de la délivrance du brevet: **24.05.2000**
(21) Numéro de dépôt: 94930239.2
(22) Date de dépôt: 07.10.1994
(51) Int. Cl.: A61K 31/70, A61K 31/35, A61K 31/00

(54) **COMPOSITION POUR LE TRAITEMENT OU LA PREVENTION DE L'HERPES**
ZUSAMMENSETZUNG ZUR BEHANDLUNG ODER VORBEUGUNG VON HERPES
COMPOSITION FOR THE TREATMENT OR PREVENTION OF HERPES

(30) Priorité: 07.10.1993 FR 9311951
(43) Date de publication de la demande: 24.07.1996
(73) Titulaire: Bidel, Christian-Georges, 06400 Cannes (FR); Poirson, Jean, 06110 Le Cannet (FR)
(72) Inventeur: Bidel, Christian-Georges, 06400 Cannes (FR); Poirson, Jean, 06110 Le Cannet (FR)
(74) Mandataire: Ahner, Francis
(86) Numéro de dépôt international: FR9401176
(87) Numéro de publication internationale: WO9509635

(56) Documents cités:
- WO-A-92/15315
- US-A- 4 049 798
- J.MED. VIROL., vol.15, no.1, 1985 pages 71 - 79 T.N. KAUL ET AL. 'Antivaral effect of flavonoids on human viruses.'
- EXPERIENTIA, vol.41, no.7, 1985 pages 930 - 931 I. MUSCI ET AL. 'Inhibition of virus multiplication and alternation of cyclic AMP level in cell cultures by flavonoids.'

## Description

La présente invention concerne la préparation de nouvelles compositions destinées à la prévention et/ou au traitement des poussées d'herpès.

L'herpès est une affection très contagieuse provoquée par un virus Herpes simplex de type I ou II, dont l'unique réservoir est l'homme.

Elle peut atteindre la peau ou les muqueuses et se propage par simple contact, augmentant d'autant le nombre des individus exposés aux risques liés à cette maladie. La primo-infection herpétique est surtout observée chez les enfants, où elle se manifeste par des lésions vésiculaires, souvent rompues, très douloureuses, prurigineuses, atteignant souvent la bouche et la langue.

Outre son aspect gênant (douleurs, contraintes, esthétique), l'herpès est une maladie pouvant avoir des conséquences graves, en particulier pour les personnes fragiles, les femmes enceintes, les nouveaunés, ainsi que dans les cas d'affection oculaire.

Actuellement, il n'existe pas de traitement réel contre cette maladie virale. Certaines thérapeutiques peuvent apporter un soulagement durant les éruptions et éventuellement en réduire la durée mais n'empêchent pas leur évolution. Il en est ainsi du plus répandu d'entre eux l'aciclovir commercialisé par les Laboratoires Wellcome sous le nom de Zovirax ®.

Les flavonoïdes sont des polyphénols comportant un squelette de base à 15 atomes de carbone résultant de la fusion de deux unités aromatiques dont l'une dérive du benzène et l'autre du phénylpropane. Les plus répandues sont les anthocyanes, les flavones et les flavonols qui peuvent exister soit sous forme libre, soit lié à un ou plusieurs sucres (hétérosides).

Les bioflavonoïdes ou citroflavonoïdes, ou complexe vitaminique P ont été connus tout d'abord pour leur activité favorable sur les vaisseaux sanguins, par diminution de la pennéabilité et de la fragilité capillaire, et leurs activité synergique avec l'acide ascorbique. Ils dérivent des flavones, de formule générale I :

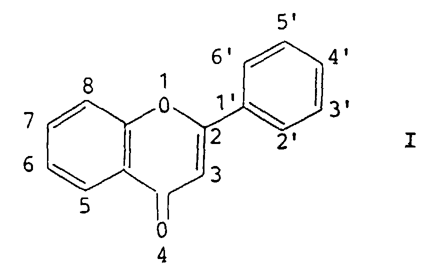

Des dérivés hémisynthétiques de flavones ont été préparés par exemple dans les demandes EP 290915 ou EP 319412. Différents groupes de dérivés flavoniques ont été utilisés pour d'autres applications pharmaceutiques que celles initialement décrites.

EP 505937 décrit la préparation de dérivés carbamoylés et sulfatés des flavones, et leur utilisation pour leurs propriétés antiélastases.

Des dérivés des flavones ont été proposés pour leur activité dans le traitement la lithiase cystinique, comme antinéoplasique, relaxant des muscles lisses ou dans le traitement du diabète.

EP 183169 concerne des halogènohydroxy-flavones et leur utilisation dans des médicaments pour le traitement de l'hyperthyroïdie.

Dans la demande EP 450588, des dérivés du type 3-oxo alkylflavone sont préparés et leur action hypocholestérolémiante est rapportée.

Des compositions antivirales contenant une combinaison d'un agent antiviral et d'un glycoside pouvant dériver des flavones, permettent de diminuer les effets secondaires habituellement observés et sont décrites dans EP 312222.

La demande EP 19081 a été déposée pour de nouveaux dérivés de flavone substitués, utilisés pour la préparation de médicaments destinés à lutter contre les Rhinovirus et les Enterovirus.

Dans EP 543555, des dérivés phosphorylés de flavonoïdes sont proposés pour leur activité en particulier sur le HIV.

De manière surprenante, la Demanderesse a mis en évidence l'activité à titre préventif et/ou curatif des poussées d'herpès, de compositions contenant des flavonoïdes non phosphorylés, et plus particulièrement des citroflavonoïdes.

C'est pourquoi la présente invention à pour objet, l'utilisation d'au moins un principe actif de la famille des flavonoïdes pour la préparation de compositions destinées au traitement et/ou à la prévention de l'herpès.

Les flavonoïdes sont les flavonoïdes naturels et plus particulièrement les citroflavonoïdes et les dérivés hémisynthétiques de flavones.

De manière avantageuse, on utilise les hétérosides de flavonoïdes pour la préparation des compositions. Les flavonosides comportent un ou plusieurs oses, pouvant notamment être le rutinose, le mannose, le glucose, etc.

De manière préférée, les flavonoïdes utiles selon l'invention sont substitués en 4' par un radical méthoxy.

En effet, une efficacité dans la prévention et/ou le traitement des crises d'herpès est observée pour des compositions qui contiennent de la diosmine ou de l'hespéridine. Des résultats intéressants sont également obtenus avec des compositions qui contiennent une association de diosmine et d'hespéridine, ce qui suggère l'existence d'une synergie des activités de ces deux composés.

En revanche, des compositions qui contiennent de la rutine sont dépourvues d'effet dans les applications selon l'invention ; la molécule de rutine est substituée par un hydroxy en position 4'.

La diosmine et/ou l'hespéridine ont été administrées chez l'homme depuis plusieurs années dans d'autres indications. Il s'agit de composés bien tolérés, provoquant peu d'effets secondaires, et dont la formulation galénique est maîtrisée. Aux doses couramment utilisées comme protecteurs veineux, ces composés sont actifs pour le traitement et/ou la prophylaxie des crises d'herpès. En particulier, les compositions selon l'invention peuvent être formulées sous forme de doses unitaires contenant de 100 à 900 mg de diosmine.

Selon l'un des aspects de l'invention, les flavonoïdes servent à la préparation de compositions pharmaceutiques destinées à la prévention et/ou au traitement des poussées d'herpès.

Selon un autre aspect, les compositions selon l'invention sont a usage cosmétique, et/ou dermo-cosmétique.

Les compositions préparées selon l'invention permettent d'empêcher le développement de la crise d'herpès, de type I ou II.

Elles en réduisent considérablement les effets, en particulier la contagion très importante lors de la phase secondaire pendant laquelle éclate le bouquet herpétique, les conséquences graves comme celles résultant d'affections oculaires ainsi que sur le confort des personnes atteintes.

La Demanderesse a montré que certains flavonoïdes, seuls ou en association, sont très efficaces pour le traitement de l'herpès et cela, à divers stades de l'évolution de l'affection :
- un usage préventif élimine toute manifestation de l'herpès,
- un usage curatif précoce permet d'arrêter une crise en cours de développement,
- un usage curatif tardif permet de raccourcir les délais de cicatrisation et d'éliminer les effets douloureux.

Les compositions contiennent en outre des véhicules et excipients pharmaceutiquement et/ou cosmétologiquement acceptables, connus de l'homme du métier.

Les compositions préparées selon l'invention peuvent contenir des excipients adaptés à une formulation pour administration par voie orale, telle que gouttes, comprimés, gélules. De manière avantageuse, la composition est formulée en dose unitaire contenant de 20 mg à 800 mg d'hespéridine.

Les compositions selon l'invention peuvent être sous une forme adaptée à l'administration par voie locale, par exemple sous forme de crème, pommade, lotion, collyre, ou de stick solide.

Dans les utilisations selon l'invention, les flavonoïdes peuvent être associés à d'autres principes actifs susceptibles de renforcer leur action.

Les exemples qui suivent sont destinés à illustrer l'invention sans aucunement en limiter la portée.

### Exemple 1

Un homme de 43 ans, souffrant depuis plusieurs années d'herpès génital chronique avec poussées de fréquence mensuelle environ, n'a constaté aucune crise pendant une période de 5 mois au cours de laquelle il absorbait par voie orale 3 prises quotidiennes de 450 mg de diosmine et 50 mg d'hespéridine sous forme de dragées (Daflon ® 500 commercialisé par les Laboratoires Servier). Les crises d'herpès se sont manifestées à nouveau dès l'arrêt du traitement.

### Exemple 2

L'absorption par le sujet mentionné dans l'exemple 1, dès les premières manifestations de la poussée d'herpès (picotements, sensation de brûlure), de 5 prises par voie orale de 900 mg de diosmine et 100 mg d'hespéridine (2 dragées de Daflon ® 500) réparties sur une durée de 36 heures, a totalement arrêté la crise, sans apparition des lésions habituelles (bouquet herpétique). Ce résultat a été vérifié de manière constante pendant une période de 14 mois.

### Exemple 3

La substitution dans l'exemple 2 de 900 mg de diosmine et 100 mg d'hespéridine par 300 mg de diosmine et 300 mg d'hespéridine (2 dragées de Daflon ® 375 au lieu de Daflon ® 500) a abouti au même résultat, à savoir l'arrêt du développement d'une poussée d'herpès naissante.

### Exemple 4

La substitution dans l'exemple 2 de 900 mg de diosmine et 100 mg d'hespéridine par 450 mg de diosmine et 50 mg d'hespéridine (1 dragée de Daflon ® 500 au lieu de 2) n'a pas permis d'aboutir au même résultat, la poussée d'herpès ayant évolué vers l'apparition de vésicules puis de lésions, quoique de manière moins virulente que lorsqu'aucun traitement n'a été mis en oeuvre. Ce résultat ayant été confirmé à plusieurs occasions, il semblerait, en comparant ces conditions de traitement avec celles de l'exemple 3, que l'association de diosmine et d'hespéridine présente un caractère de synergie dans le traitement des crises d'herpès.

### Exemple 5

La substitution dans l'exemple 2 de 900 mg de diosmine et 100 mg d'hespéridine par 600 mg d'hespéridine (Hesperidin 97%, référence 29, 260-5 de Aldrich-Chimie Sarl) en dispersion aqueuse, a abouti au même résultat, à savoir l'arrêt du développement d'une poussée d'herpès naissante.

### Exemple 6

L'exemple 4 a été répété à plusieurs reprises en complétant le traitement par voie orale par un traitement locale avec une pommade contenant 5% d'hespéridine (Hesperidin 97%, référence 29, 260-5 de Aldrich-Chimie Sarl) dispersée dans une base hydrophile (Biobase des Laboratoires Bioderma). L'éruption a cessé d'évoluer après 24 heures et s'est résorbée pendant les 48 heures suivantes.

### Exemple 7

Un homme de 45 ans, souffrait depuis de nombreuses années d'herpès labial avec poussées de fréquence mensuelle environ. L'absorption par ce sujet, dès les premières manifestations de la poussée d'herpès, de 5 prises par voie orale de 900 mg de diosmine et 100 mg d'hespéridine (2 dragées de Daflon ® 500) réparties sur une durée de 36 heures, a totalement arrêté la crise, avec résorption rapide de la vésicule naissante et sans apparition des lésions habituelles. Ce résultat a été vérifié de manière constante pendant une période de 14 mois.

### Exemple 8

La substitution dans l'exemple 5 de 900 mg de diosmine et 100 mg d'hespéridine par 600 mg d'hespéridine (Hesperidin 97%, référence 29, 260-5 de Aldrich-Chimie Sarl) en dispersion aqueuse, a abouti au même résultat, à savoir l'arrêt du développement d'une poussée d'herpès naissante.

### Exemple 9

Une femme de 47 ans souffrait depuis de nombreuses années d'herpès lombaire avec poussées de fréquence mensuelle environ. L'absorption par ce sujet dès les premières manifestations de la poussée d'herpès, de 5 prises par voie orale de 900 mg de diosmine et 100 mg d'hespéridine (2 dragées de Daflon ® 500) réparties sur une durée de 36 heures, a totalement arrêté la crise, avec résorption rapide des vésicules naissantes et sans apparition des lésions habituelles. Ce résultat a été vérifié de manière constante pendant une période de 14 mois.

### Exemple 10

Une femme de 40 ans, sujette à des crises d'herpès à l'oeil droit n'a plus fait l'objet de cette affection aux conséquences potentielles graves en absorbant, dès les premières manifestations de brûlure, 5 prises par voie orale de 900 mg de diosmine et 100 mg d'hespéridine (2 dragées de Daflon ® 500) réparties sur une durée de 36 heures. Ce résultat a été vérifié de manière constante pendant une période de 26 mois.

## Revendications

1. Utilisation d'au moins un principe actif de la famille des flavonoïdes, sous forme d'un hétéroside, pour la préparation de compositions destinées au traitement et/ou à la prévention de l'herpès.

2. Utilisation selon la revendication 1, caractérisée en ce que les flavonoïdes sont des citroflavonoïdes ou leurs dérivés.

3. Utilisation selon l'une des revendications 1 ou 2, caractérisée en ce que la composition contient de la diosmine.

4. Utilisation selon l'une des revendications 1 à 3, caractérisée en ce que la composition contient de l'hespéridine.

5. Utilisation selon l'une des revendications 1 à 4, caractérisée en ce qu'on utilise une association de diosmine et d'hespéridine.

6. Utilisation selon l'une des revendications 1 à 5, caractérisée en ce que la composition est une composition dermo-cosmétique.

7. Utilisation selon l'une des revendications 1 à 5, caractérisée en ce que les compositions sont des compositions pharmaceutiques.

8. Utilisation selon l'une des revendications 1 à 5 et 7, caractérisée en ce que la composition comprend en outre des excipients adaptés pour la voie orale.

9. Utilisation selon la revendication 8, caractérisée en ce que la composition est formulée sous forme de dose unitaire contenant de 20 mg à 800 mg d'hespéridine.

10. Utilisation selon l'une des revendications 1 à 7, caractérisée en ce que la composition est sous une forme adaptée à l'administration par voie locale.

## Claims

1. Use of at least one active principle of the flavonoid family, in the form of a heteroside, for the preparation of compositions intended for the treatment and/or prevention of herpes.

2. Use according to Claim 1, characterized in that the flavonoids are citroflavonoids or their derivatives.

3. Use according to either of Claims 1 and 2, characterized in that the composition contains diosmin.

4. Use according to one of Claims 1 to 3, characterized in that the composition contains hesperidin.

5. Use according to one of Claims 1 to 4, characterized in that a combination of diosmin and hesperidin is used.

6. Use according to one of Claims 1 to 5, characterized in that the composition is a dermocosmetic composition.

7. Use according to one of Claims 1 to 5, characterized in that the compositions are pharmaceutical compositions.

8. Use according to one of Claims 1 to 5 and 7, characterized in that the composition additionally comprises excipients suited to the oral route.

9. Use according to Claim 8, characterized in that the composition is formulated in the form of a unit dose containing from 20 mg to 800 mg of hesperidin.

10. Use according to one of Claims 1 to 7, characterized in that the composition is in a form suited to local administration.

## Patentansprüche

1. Verwendung mindestens eines Wirkstoffs aus der Familie der Flavonoide in Form eines Heterosides zur Herstellung von Zusammensetzungen zur Behandlung von oder Vorbeugung gegen Herpes.

2. Verwendung nach Anspruch 1, dadurch gekennzeichnet, daß die Flavonoide Zitroflavonoide oder deren Derivate sind.

3. Verwendung nach einem der Ansprüche 1 oder 2, dadurch gekennzeichnet, daß die Zusammensetzung Diosmin enthält.

4. Verwendung nach einem der Ansprüche 1 bis 3, dadurch gekennzeichnet, daß die Zusammensetzung Hesperidin enthält.

5. Verwendung nach einem der Ansprüche 1 bis 4, dadurch gekennzeichnet, daß man eine Assoziation von Diosmin und Hesperidin verwendet.

6. Verwendung nach einem der Ansprüche 1 bis 5, dadurch gekennzeichnet, daß die Zusammensetzung eine dermokosmetische Zusammensetzung ist.

7. Verwendung nach einem der Ansprüche 1 bis 5, dadurch gekennzeichnet, daß die Zusammensetzungen pharmazeutische Zusammensetzungen sind.

8. Verwendung nach einem der Ansprüche 1 bis 5 oder 7, dadurch gekennzeichnet, daß die Zusammennetzung Zusätze zur oralen Verabreichung enthält.

9. Verwendung nach Anspruch 8, dadurch gekennzeichnet, daß die Zusammensetzung als Einheitsdosis mit Gehalt an 20 mg bis 800 mg Hesperidin formuliert ist.

10. Verwendung nach einem der Ansprüche 1 bis 7, dadurch gekennzeichnet, daß die Zusammensetzung zu lokaler Verabreichung formuliert ist.
